# EUROPEAN PATENT SPECIFICATION

(11) **EP 2 879 564 B1**
(45) Date of publication and mention of the grant of the patent: **01.06.2016**
(21) Application number: 13752853.5
(22) Date of filing: 30.07.2013
(51) Int. Cl.: A61B 3/103

(54) **APPARATUS FOR DETECTING OCULAR DEFECTS**
VORRICHTUNG FÜR DEN NACHWEIS VON AUGENLEIDEN
APPAREIL POUR DÉTECTER DES DÉFAUTS OCULAIRES

(30) Priority: 31.07.2012 IT TV20120151
(43) Date of publication of application: 10.06.2015
(73) Proprietor: Adaptica S.r.l., 35129 Padova (IT)
(72) Inventor: MENEGHINI, Gianluigi, I-35129 Padova (IT); ANGI, Mario, I-35129 Padova (IT)
(74) Representative: De Bortoli, Eros
(86) International application number: PCT/EP2013/065969
(87) International publication number: WO 2014/020013

(56) References cited:
- DE-A1- 3 629 695
- US-A- 4 834 528
- US-A1- 2011 157 550

## Description

The present invention relates to an apparatus for detecting ocular defects.

The use of apparatus for detecting ocular defects, for example focusing defects or aberrations of the field of vision, is widely known.

These apparatus, known as refractometers or skiascopes, optically conjugate the ocular fundus with a light emitting device configured to illuminate the retina with a beam of light, for example infrared light.

The light reflected by the retina is picked up, through the central portion of the pupil, by an imaging device that photographs the ocular fundus illuminated by the light emitted by the light emitting device.

Patent US3879113 describes a refractometer particularly suitable for detecting astigmatism, according to which the patient's retina is illuminated by a light source formed by the end of an optical fibre, positioned inside the optical field of the objective of an imaging device.

This apparatus has a relatively complex structure, bulky and costly to produce on an industrial scale.

Patent US4834528 describes another apparatus for detecting occular defects.

In other prior art apparatus the ocular fundus is illuminated by an infrared light beam deviated by a beam splitter device, separate from the photographic objective and positioned along the optical path between a plurality of light sources and the ocular fundus, to deviate the light coming from these light sources towards the patient's eyes.

These apparatus of conventional type also have some drawbacks.

A beam splitter device positioned along the optical path of the light towards the patient's eyes interferes optically with the light beam reaching the photographic device, reducing the contrast and the quality of the photographs of the ocular fundus.

The precision in detecting any ocular defects is thus considerably reduced, with high percentages of false positives or negatives.

The structure of these apparatus is also relatively complex, bulky and, consequently, costly to produce on an industrial scale.

The main aim of the present invention is to provide an apparatus for detecting ocular defects which enables the problems of prior art, indicated above, to be solved.

Within this aim, an object of the present invention is to provide an apparatus that enables rapid detection of any ocular defects, with relatively high quality of the photographic images and measurement precision.

A further object of the present invention is to provide an apparatus characterized by noteworthy compactness, simple construction and limited overall dimensions.

A further object of the present invention is to provide an apparatus that is easy to produce on an industrial scale, at competitive costs.

This aim and these objects, as well as other objects that will be apparent from the description below and from the accompanying drawings, are achieved, according to the invention, by an apparatus for detecting ocular defects according to claim 1, proposed below.

Further characteristics and advantages of the apparatus according to the invention will be more apparent with reference to the description given below and to the accompanying figures, provided purely for explanatory and non-limiting purposes, wherein:

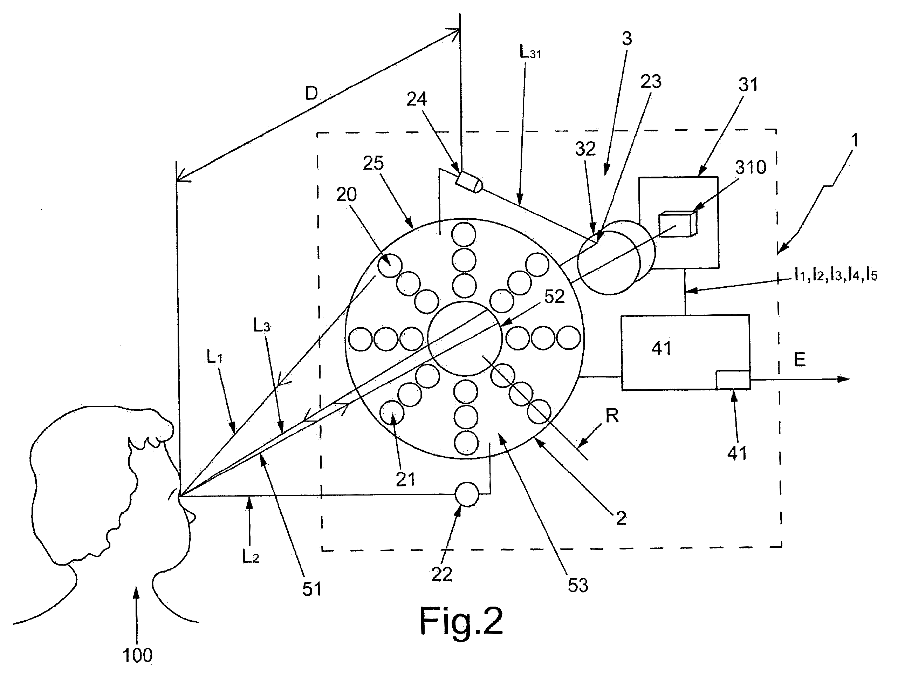

- Figs. 1-2 schematically show the apparatus according to the invention in a possible embodiment thereof;

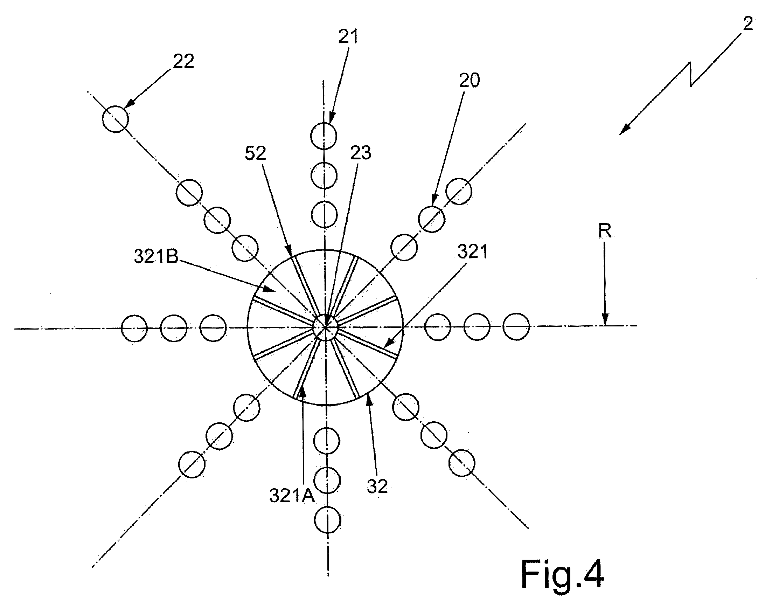

- Figs. 3, 3a, 4 schematically show some embodiments of a lighting device, included in the apparatus according to the invention; and
- Figs. 5-6 schematically show examples of detecting ocular defects by means of the apparatus according to the invention.

With reference to the aforesaid figures, the present invention relates to an apparatus 1 for detecting ocular defects in a patient 100, for example to a refractometer or a skiascope.

The apparatus 1 comprises lighting means 2 and imaging means 3.

The lighting means 2 are configured to project light beams L₁, L₂, L₃ to illuminate the ocular fundus of the patient's eyes.

Preferably the light beams L₁, L₂, L₃ are infrared light beams.

Advantageously, the lighting means 2 radiate light beams L₁, L₂, L₃ towards both of the patient's eyes (binocular illumination).

The imaging means 3 comprise detection means 31 configured to receive light L_{R} reflected by the ocular fundus, at a receiving surface 310, to be able to acquire photographic images I₁, I₂, I₃, I₄, I₅ of the ocular fundus of the patient's eyes.

The photographic images I₁, I₂, I₃, I₄, I₅ are advantageously focused substantially at the level of the pupil. Each of these shows a certain distribution of the light reflected by the ocular fundus (known as "crescent of light").

Advantageously, the imaging means 3 receive the light reflected by both of the patient's eyes (binocular imaging).

The imaging means 3 can be formed by a digital imaging device. The detection means 31 can advantageously comprise CCD or C-MOS sensors, positioned at the receiving surface 310.

The imaging means 3 advantageously comprise an objective group 32, positioned upstream of the receiving surface 310, with reference to the optical path travelled by the light L_{R}.

The objective group 32 has an optical axis 51, directed towards the patient's eyes, and an opening 52, centred on the optical axis 51, which defines the optical field of the same objective group.

The optical path of the light L_{R}, reflected by the ocular fundus and collected by the receiving surface 310, through the patient's pupils, extends along the optical axis 51, passing through the objective group 32.

The objective group 32 advantageously comprises a system of lenses conjugated with the patient's pupil and having a focal distance D comprised between 0.3 m and 2 m.

Preferably, the objective group 32 has a focal distance D of 1 m.

This latter solution is particularly advantageous, as it makes it possible to obtain good definition of the crescent of light, with an optimal level of luminous power emitted by the lighting means 2.

This distance also corresponds to an optical distance of one dioptre. This simplifies processing of the photographic images I₁, I₂, I₃, I₄, I₅ to obtain data indicative of ocular defects.

The apparatus 1 also comprises a control unit 4 configured to control the operation of the lighting means 2 and of the imaging means 3 by providing appropriate control signals C.

The control unit 4 can advantageously be formed by a digital processing device, such as a microcontroller.

The control unit preferably comprise digital processing means 41 that are capable to store and execute software programmes, modules or procedures.

Preferably, the control unit 4 is arranged to receive images I₁, I₂, I₃, I₄, I₅ of the ocular fundus, acquired by the imaging means 3.

The processing means 41 are capable to process the information so received and to automatically provide information E relating to the presence of any ocular defects and/or relating to other characteristics of the ocular fundus, based on the images received.

The control unit 4 can be operatively associated with a man-machine interface (not shown) for entering manual commands or for the execution of configuration or programming operations.

According to the invention, the lighting means 2 comprise at least one lighting group 20 comprising a plurality of first extended light sources 21 configured to emit light L₁.

Within the scope of the present invention, a light source capable of projecting a light spot of non-negligible width (for example of around 5 mm) is considered as an "extended" or "non-point source".

Preferably, the light sources 21 are formed by first infrared LED devices.

According to the invention, the light sources 21 are positioned at the opening 52 of the objective group 32, in a space region external to this opening.

In other words, the light sources 21 are positioned externally to the optical field of the objective group 32.

The light sources 21 are positioned in predefined positions along a corresponding reference axis R, at different distances with respect to the optical axis 51.

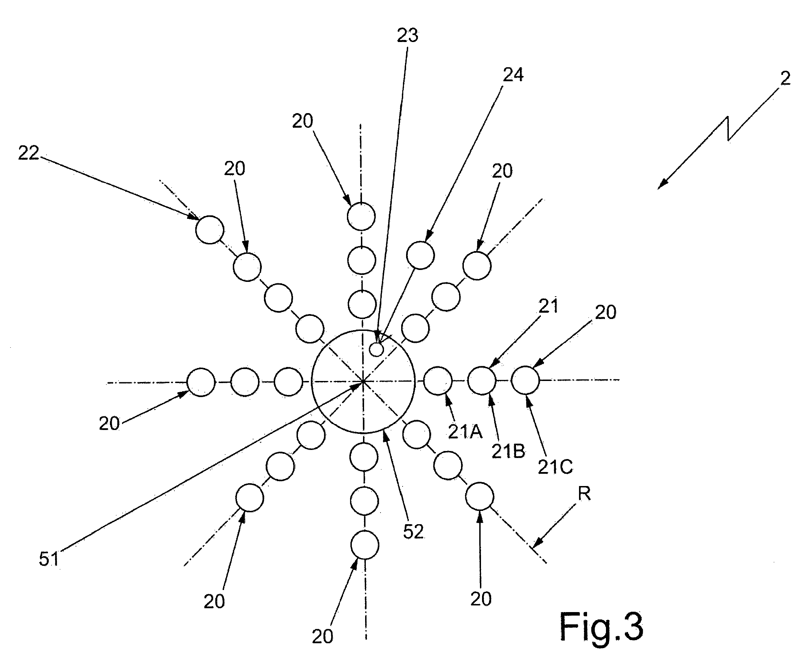

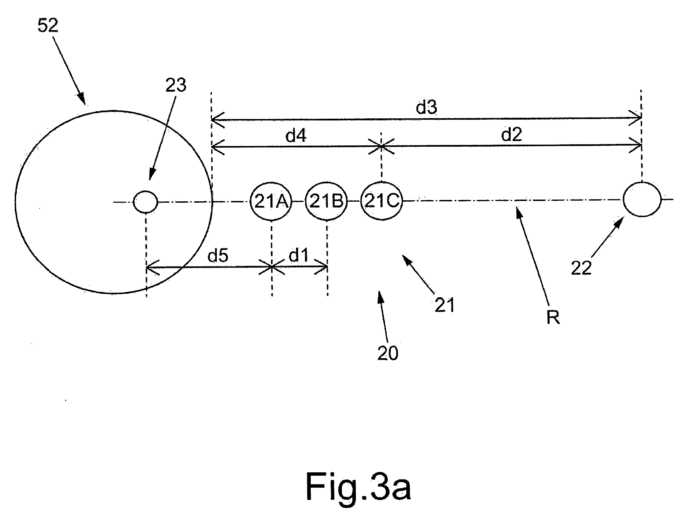

Preferably, the light sources 21 are equidistant from one another. As an example, the relative distance *d1* between two subsequent light sources 21 (e.g. the light sources 21A, 21B) along the rereference axis R may be about 6mm (Fig. 3a).

Preferably, the light sources 21 are positioned on a common reference plane 53, substantially perpendicular to the optical axis 51.

The reference plane 53 can be defined by a support 25 (for example a board for electronic printed circuits) in fixed position with respect to the objective group 32.

The reference axis R, along which the light sources 21 are positioned, lies on this reference plane 53 and is oriented radially with respect to the optical axis 51 (and the opening 52).

According to the invention, the light sources 21 can be consecutively activated by the control unit 4 according to at least a first activation time sequence, which can advantageously be determined based on their position.

For example, the control unit 4 can consecutively activate the light sources 21 starting from the one closest to the optical axis 51, or vice versa.

The first activation time sequence comprises a series of activation instants that are calculated by the control unit 4 as a function of the relative distance *d1* between the light sources 21.

Being consecutively activated according to the aforesaid first activation time sequence, the light sources 21 are controlled by the control unit 4 so as to be capable to emulate the illumination produced by a same extended or non-point light source in translatory movement along the reference axis R through a space external to the optical field of the objective group 32.

In other words, by activating the light sources 21 according to the aforesaid first activation time sequence, the ocular fundus of the patient's eyes is illuminated as if it were illuminated by a same extended light source in movement along the reference axis R, according to a predefined direction of movement.

When the light sources 21 are activated according to the aforesaid first activation sequence, the imaging means 3 acquire first images I₁ of the ocular fundus of the patient's eyes.

The imaging means 3 acquire a sequence of first images I₁, each of which photographs the ocular fundus, illuminated by a corresponding light source 21, activated according to the aforesaid first activation time sequence.

Advantageously, the processing means 41 process the images I₁ received from the imaging means 3 and provide information E relating to the presence of any ocular defects based on the data obtained from the aforesaid images.

By activating the light sources 21 of the lighting group 20, according to the aforesaid first activation sequence, it is possible to obtain, rapidly and effectively, information regarding the presence of focusing defects of the eye, for example myopia and hypermetropia.

Preferably, the processing means 41 execute a processing procedure that comprises the following steps:
- activating the light sources 21 according to said first activation time sequence;
- receiving the sequence of images I₁ from the imaging means 3;
- identifying the front-edge of the crescent of light in the sequence of images I₁;
- calculating data related to the position and moving speed of the front-edge of the crescent of light in the sequence of images I₁;
- providing information E related to the type of sight defect identified (for example hypermetropia) and the extent (measured in dioptres) thereof on the base of the data calculated at the previous steps.

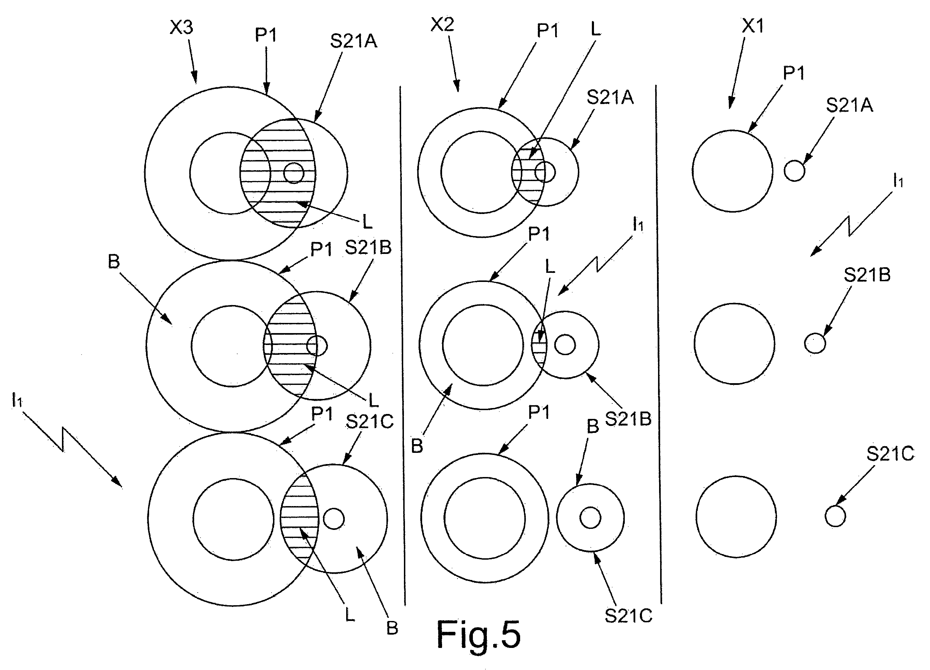

With reference to Figs. 3 and 5, an example of detecting focusing defects of the eye is shown.

Let us suppose that the ocular fundus of the patient's eyes is illuminated by a lighting group 20 comprising three light sources 21A, 21B, 21C, which are activated by the control unit 4, according to an activation time sequence that consecutively activates them starting from the one closest to the optical axis 51.

Fig. 5 shows a sequence X1 of images I₁, relating to the ocular fundus of a healthy patient's eyes, photographed by the imaging means 3 when the ocular fundus is illuminated by the light sources 21A, 21B, 21C, activated according to the aforesaid activation time sequence.

In each image I₁ of the ocular fundus, it is possible to identify the projection (dark area) P1 of the opening of the objective group 21 and the projection (light spot) S_{21A}, S_{21B}, S_{21C} of the light radiation L₁ emitted by each of the light sources 21A, 21B, 21C.

It can be observed that there are no overlapping areas L between the areas P1 and the light spots S_{21A}, S_{21B}, S_{21C} produced by the light sources 21A, 21B, 21C.

In general, this lack of overlapping areas L indicates the absence of sight defects.

Fig. 5 shows another sequence X2 of images I₁, relating to the ocular fundus of a patient's eyes with a slight focusing defect of the eye.

Also in this case, the images I₁ of the sequence X2 are photographed by the imaging means 3 when the ocular fundus is illuminated by the light sources 21A, 21B, 21C, activated according to the aforesaid activation time sequence.

It can be observed how, in this case, for each image I₁, the areas P1 and the light spots S_{21A}, S_{21B}, S_{21C} are more extensive, compared to a healthy patient. A blur B is present due to the fact that focusing of the light radiation received does not occur at retina level.

In the images of the sequence X2, it can be observed how some overlapping areas L are present between the areas P1 and the light spots S_{21A}, S_{21B}, S_{21C}.

By measuring and correlating with one another the overlapping areas L, which can be observed from the images I₁ of the sequence X2, and appropriately processing the data thus obtained (in particular the data related to the position and moving speed of the the front-edge of the crescent of light), the processing means 41 are advantageously able to provide information E relating to the type of sight defect identified (for example hypermetropia) and the extent (measured in dioptres) thereof.

Finally, Fig. 5 shows a further sequence X3 of images I₁, relating to the ocular fundus of a patient's eyes with a marked focusing defect of the eye.

It is evident how extensive overlapping areas L are present between the areas P1 and the light spots S_{21A}, S_{21B}, S_{21C}.

Also in this case, the processing means 41 are advantageously able to provide information E relating to the type of sight defect identified and to the extent thereof, based on the data calculated from the images of the ocular fundus.

To ensure that the sequences of images, acquired by the imaging means 3, comprise a sufficiently high number of samples, the lighting group 20 preferably comprises at least three light sources 21.

In this way, it is possible to ensure relatively high measurement precision in detecting focusing defects that are not particularly extensive, for example up to +/- 6 dioptres.

In some preferred embodiments of the present invention, the lighting means 2 preferably comprise at least a second extended light source 22, configured to emit light L₂.

Preferably, the light source 22 is formed by an infrared LED.

The light source 22 is positioned at the opening 52 of the objective group 32, in a space region external to the same opening 52.

Preferably, the light source 22 is positioned along the same reference axis R, along which the light sources 21 are positioned.

Preferably, the light source 22 is positioned at a relatively long distance from the optical axis 51, with respect to the light sources 21.

Preferably, the distance *d2* of the second light source 22 from the closest first light source 21C (moving along a reference axis R) is given by *d2* >=(2,5 * *d1*), where *d1* is the relative distance between two subsequent first light sources 21 (e.g. the light sources 21A, 21B) along the same reference axis R (Fig. 3a).

Preferably, the distance *d3* of the light source 22 from the opening 52 (moving along a same reference axis R) is given by *d3* >=(5 * *d4*), where *d4* is the distance from the opening 52 of the first light source 21C, which is closest to the light source 22, along the same reference axis R (Fig. 3a).

The light source 22 can advantageously be activated by the control unit 4 autonomously with respect to the light sources 21.

As an example, the light source 22 can be switched off when the light sources 21 are activated and vice versa.

The light source 22 is thus not part of the lighting groups 20 from a functional and structural point of view.

When the light source 22 is active, the imaging means 3 acquire one or more third images I₃ of the ocular fundus.

Advantageously, the processing means 41 process the images I₃ received from the imaging means 3 and provide information E relating to the presence of any ocular defects, based on the data obtained from the aforesaid images.

The light source 22 can advantageously be used if the process for detecting focusing defects of the eye, performed by sequentially activating the light sources 21 of the lighting group 20, provided a negative or unsatisfactory result.

The use of the light source 22 is particularly useful for identifying out-of-range focusing defects (for example exceeding +/- 6 dioptres) which might not be detected by sequentially activating the light sources 21, according to the operating modes described above.

Preferably, the lighting means 2 comprise a plurality of lighting groups 20, each of which comprises a plurality of light sources 21, positioned as shown above.

Advantageously, each lighting group 20 is positioned at a different angular position with respect to the optical axis 51 and identifies an angular sector, external to the optical field of the objective group 32 and centred on the optical axis 51.

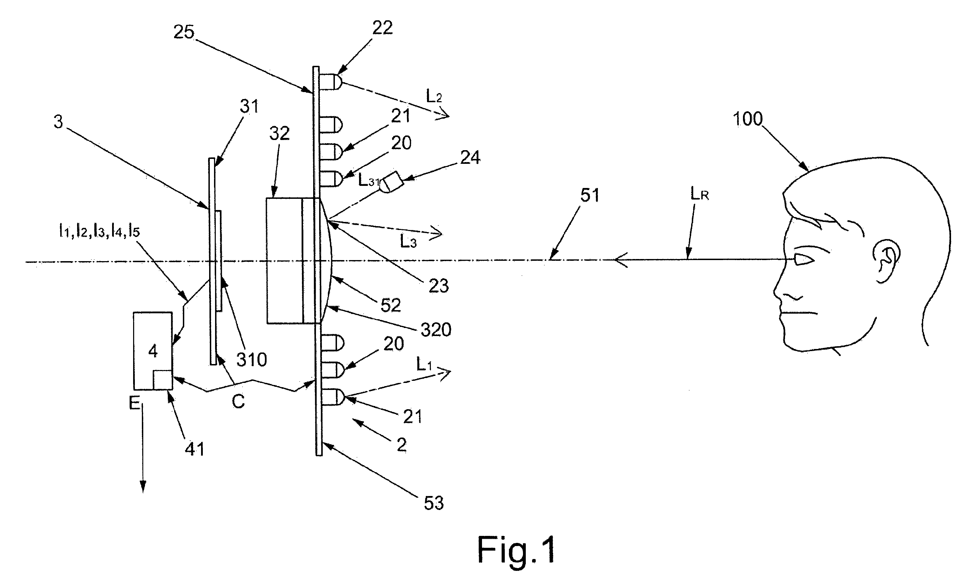

Preferably, the light sources 21 of each lighting group 20 are positioned along a corresponding reference axis R, lying on a common reference plane 53, substantially perpendicular to the optical axis 51 (Figs. 1, 3).

The reference axes R of the lighting groups 20 are preferably orientated radially with respect to the optical axis 51 (and the opening 52) and are angularly equidistant from one another, preferably to cover the whole round angle around the optical axis 51.

Advantageously, the light sources 21 of each lighting group 20 are consecutively activatable by the control unit 4 according to the aforesaid first activation time sequence.

Each of the lighting groups 20 can thus be used autonomously to detect the presence of any focusing defects of the eye, according to the operating methods illustrated above.

Preferably, the lighting groups 20 are also consecutively activatable by the control unit 4 according to a second activation time sequence.

Activation of each lighting group 20 causes illumination of one or more of the light sources 21 thereof.

For each lighting group 20, the light sources 21 positioned in the same position with respect to the optical axis 51, along the corresponding reference axis R, are advantageously activated. For example, with reference to Fig. 3, for each lighting group 20, all the light sources 21A, 21B, 21C could be activated simultaneously, or only the light source 21B could be activated, and so forth.

The second activation time sequence can advantageously be calculated by the control unit 4 based on the angular position of the lighting groups 20 with respect to the optical axis 51.

For example, the control unit 4 can consecutively activate the lighting groups 20, starting from a certain angular reference position, in clockwise or counter-clockwise direction.

Being consecutively activated according to the aforesaid second activation time sequence, the lighting groups 20 are controlled by the control unit 4 so as to be capable to emulate the illumination produced by a same extended light source in rotational movement around the optical axis 51, in a region external to the optical field of the objective group 32.

In other words, by consecutively activating the lighting groups 20, according to the aforesaid second activation time sequence, the ocular fundus of the patient's eyes is illuminated as if it were illuminated by a same extended light source in rotation around the optical axis 51, according to a predefined direction of movement, to cover the round angle.

When the lighting groups 20 are activated according to the aforesaid second activation sequence, the imaging means 3 acquire second images I₂ of the ocular fundus of the patient's eyes.

The imaging means 3 acquire a sequence of second images I₂, each of which photographs the ocular fundus, illuminated by one of the lighting groups 20, activated according to the aforesaid second activation time sequence.

Advantageously, the processing means 41 process the images I₂ received from the imaging means 3 and provide information E relating to the presence of any ocular defects, based on the data obtained from the aforesaid images.

By activating the lighting groups 20, according to the aforesaid second activation sequence, it is possible to obtain, rapidly and effectively, information regarding the presence of ocular defects that may affect the curvature of the eye, for example astigmatism.

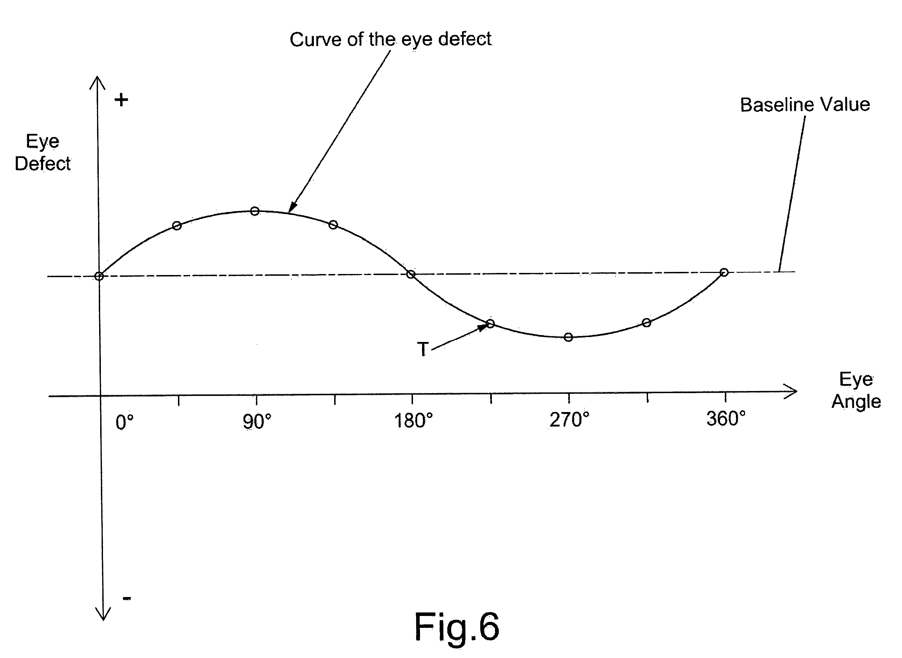

With reference to Fig. 6, an example of detection of ocular defects is shown.

Each of the lighting groups 20, activated according to the second activation time sequence, illuminates the ocular fundus from a corresponding angular position around the optical axis 51.

The corresponding image I₂ of the ocular fundus, photographed by the imaging means 3, makes it possible to obtain a measurement of the eye defect at a meridian of the eye, corresponding to the angular position of the activated lighting group.

The acquisition of a sequence of second images I₂, each of which photographs the ocular fundus, illuminated by one of the lighting groups 20, activated according to the aforesaid second activation sequence, makes it possible to obtain a series of measurement samples T, indicative of the extent of the eye defect, at certain meridians of the eyes.

The measurement samples T allow reconstruction of the curve of the eye defect as a function of the ocular angle (Fig. 6).

The curve of the eye defect can, for example, be:
- a straight line that extends along the axis of baseline value and that coincides with the abscissa axis: this indicates a substantial absence of ocular defects;
- a straight line that extends along the axis of baseline value and that does not coincide with the abscissa axis: this indicates a substantial absence of curvature defects (for example astigmatism) but indicates the presence of focusing defects;
- a substantially sinusoidal curve along the axis of baseline value: this indicates the presence of astigmatism;
- a non-sinusoidal curve along the axis of baseline value: this indicates the presence of more complex spherical aberrations.

Obviously, information of different type, with respect to those listed above, relating to the presence and extent of ocular defects, can be obtained by reconstructing the curve of the eye defect.

Preferably, the lighting means 2 comprise eight lighting groups 20, whose light sources 21 are advantageously positioned along corresponding reference axes R.

These reference axes R preferably lie on a common reference plane 53, substantially perpendicular to the optical axis 51 of the objective group 32, are oriented radially with respect to the optical axis 51 and are angularly equidistant from one another by an angle of 45°.

This solution enables the curve of the eye defect to be reconstructed with relatively high precision. In fact, it is possible to obtain a relatively high number of measurement samples T, above all in relation to meridians of the eye, for example the horizontal and vertical meridian of the eye (respectively 0° and 90°) at which the curvature defects are typically more frequently present.

In some embodiments of the present invention, the lighting means 2 comprise at least a third extended light source 23 configured to emit light L₃.

The light source 23 is advantageously positioned internally to the optical field defined by the opening 52 of objective group 32.

According to a variant of embodiment, the light source 23 is obtained by projecting on a lens 320 of the objective group 32 a light beam L₃₁, in turn emitted by a fourth light source 24 (for example an infrared LED), positioned at the opening 52, in a space region external thereto.

In this case, the light source 23 is a virtual light source, positioned substantially at the optical axis 51, which illuminates the ocular fundus due to the deviation implemented by the surface of the lens 320 on the light beam L₃₁ that comes from a position external to the optical field of the objective group 32.

This solution is advantageous given that the optical field of the objective group 32 is free of obstacles, notwithstanding the fact that the light source 23 is visualized internally thereto.

Moreover, no bulky beam splitters are used to deviate the light beam L₃₁.

According to a further variant of embodiment, the light source 23 is formed by a light emitting device, for example an infrared LED, mounted on a supporting frame 321 that extends through the opening 52 of the objective group 32.

Preferably, the supporting frame 321 has a spoke-shaped structure configured to maintain the light source 23 in central position, substantially at the optical axis 51.

The supporting frame 321 advantageously comprises a plurality of supporting elements 321A.

Preferably, the supporting elements 321A are relatively thin and are positioned along the bisectors of the angular sectors 321B, each of which is defined by a pair of consecutive reference axes R.

In this way, the supporting frame 321 does not cause, in the optical path towards the receiving surface 310, significant reductions of the luminosity of the reflected light L_{R} or significant distortions of the images of the ocular fundus.

Preferably, the supporting frame 321 comprises eight supporting elements 321A. This enables any disturbance introduced in the images photographed by the imaging means 3 to be made uniform.

The light source 23 can advantageously be activated by the control unit 4 autonomously with respect to the light sources 21, 22.

When the light source 23 is activated, the imaging means 3 acquire one or more fourth images I₄ of the ocular fundus of the patient's eyes.

Advantageously, the processing means 41 process the images I₄ received from the imaging means 3 and provide, based on the data obtained from the aforesaid images, information E relating to certain characteristics of interest of the ocular fundus, for example the luminosity at rest thereof.

Acquisition of this information is particularly useful given that it enables considerable extension of the detection range of any focusing defects (for example up to +/- 20 dioptres), with very high precisions (maximum error of less than 0.25 dioptres).

In some embodiments of the present invention, the light sources 21 of at least one lighting group 20 and at least one of the light sources 22, 23 are consecutively activatable according to at least a third activation time sequence.

According to these embodiments, the light sources 21 of a lighting group 20 and at least one of the light sources 22, 23 form an array of light sources activatable according to the aforesaid third activation time sequence.

Advantageously, this array of light sources 21 and 22 and/or 23 extends along the reference axis R, along which the light sources 21 are positioned.

Preferably, the third activation sequence can advantageously be determined based on the position of the light sources 21 and 22 and/or 23.

For example, the control unit 4 can consecutively activate the light sources 21 and 22 and/or 23 starting from the one closest to the optical axis 51, or vice versa.

The third activation time sequence comprises a series of activation instants that are calculated by the control unit 4 as a function of the relative distances *d1* and *d2* and/or *d3* between the light sources 21 and 22 and/or 23 (Fig. 3a).

Being consecutively activated according to the aforesaid third activation time sequence, the light sources 21 and 22 and/or 23 are controlled by the control unit 4 so as to be capable to emulate the illumination produced by a same extended light source in translatory movement along the reference axis R, through the optical field of the objective group 32 and a region external thereto.

When the light sources 21 and 22 and/or 23 are activated according to the aforesaid third activation sequence, the imaging means 3 acquire fifth images I₅ of the ocular fundus of the patient's eyes.

The imaging means 3 acquire a sequence of images I₅, each of which photographs the ocular fundus illuminated by one of the light sources 21 and 22 and/or 23, activated according to the aforesaid third activation sequence.

Advantageously, the processing means 41 process the images I₅ received from the imaging means 3 and provide information E relating to the presence of any ocular defects and/or relating to other characteristics of the ocular fundus, based on the data obtained from the aforesaid images.

By activating the light sources 21 and 22 and/or 23, according to the aforesaid third activation sequence, it is possible to obtain, rapidly and effectively, information regarding the presence of focusing defects of the eye, for example myopia o hypermetropia, for a very extensive range of measurements.

Preferably, the processing means 41 execute a further processing procedure that comprises the following steps:
- activating the light sources 21 and 22 and/or 23 according to said third activation time sequence;
- receiving the sequence of images I₅ from the imaging means 3;
- identifying the front-edge of the crescent of light in the sequence of images I₅;
- calculating data related to the position and moving speed of the the front-edge of the crescent of light in the sequence of images I₅;
- obtaining information E related to the type of sight defect identified and the extent (measured in dioptres) thereof on the base of the data calculated at the previous steps.

The apparatus 1 according to the invention has considerable advantages with respect to prior art.

It enables rapid and effective acquisition of information regarding the presence of any eye defects and/or other characteristics of the ocular fundus.

The apparatus 1 ensures relatively high performance with regard to the measurement precision and the quality and resolution of the images acquired.

From an operational viewpoint, the apparatus 1 can be used with simple calibration operations and with minimum actions carried out by the operator.

The apparatus 1 has considerable flexibility of use.

It is particularly suitable for implementing detection procedures typical of the so-called "dynamic skiascopy".

However, it can also be used effectively for more conventional detection procedures, of static type.

The apparatus 1 has a very compact structure, with relatively limited weight and overall dimensions, and is easy to produce on an industrial scale, with considerable advantages in terms of limiting production costs.

## Claims

1. Apparatus (1) for detecting ocular defects in a patient (100) comprising:
- lighting means (2) configured to project a light (L₁, L₂, L₃) to illuminate the ocular fundus;
- imaging means (3) comprising detection means (31) configured to receive light reflected by the ocular fundus, at a receiving surface (310) and configured to acquire images (I₁, I₂, I₃, I₄, I₅) of the ocular fundus, said imaging means comprising an objective group (32) having an optical axis (51) directed towards the patient's eyes and an opening (52) centred on said optical axis;
- a control unit (4) configured to control the operation of said lighting means and said imaging means;
wherein said lighting means (2) comprise at least a lighting group (20) comprising a plurality of first extended light sources (21), positioned at the opening (52) of said objective group, in a space region external to said opening (52), along a reference axis (R), at different distances with respect to the optical axis (51) of said objective group; wherein said control unit (4) is configured to consecutively activate said first light sources according to at least a first activation time sequence such that the ocular fundus of the patient's eyes is illuminated as if it were illuminated by a same extended light source in movement along the reference axis (R), according to a predefined direction of movement;
wherein said imaging means (3) are configured to acquire first images (I₁) of the ocular fundus, illuminated by the light (L₁) emitted by each of said first light sources (21), when said first light sources are activated by said control unit according to said first activation time sequence;
**characterised in that** said control unit (4) is configured to:
- receive said first images (I₁) from the imaging means (3);
- identify a front-edge of a crescent of light in said first images (I₁);
- calculate data related to the position and moving speed of said front-edge of said crescent of light in said first images (I₁);
- provide information (E) related to the type and extent of a sight defect on the base of the data calculated at the previous steps.

2. Apparatus, according to claim 1, **characterised in that** said lighting group (20) comprises at least three first light sources (21), positioned along said reference axis (R).

3. Apparatus, according to one or more of the previous claims, **characterised in that** said first light sources (21) are positioned along said reference axis (R), which lies on a reference plane (53), substantially perpendicular to the optical axis (51) of said objective group, and is radially oriented with respect to the optical axis (51) of said objective group.

4. Apparatus, according to one or more of the previous claims, **characterised in that** said lighting means (2) comprise a plurality of lighting groups (20), positioned at different angular positions with respect to the optical axis (51) of said objective group, said control unit being configured to consecutively activate said lighting groups according to at least a second activation time sequence, said imaging means being configured to acquire second images (I₂) of the ocular fundus, illuminated by the light (L₁) emitted by each of said lighting groups, when said lighting groups are activated according to said second activation time sequence, said control unit being configured to receive and process said second images (I₂) and provide information (E) related to the presence of ocular defects on the base of data obtained from said second images.

5. Apparatus, according to claim 4, **characterised in that** said lighting means (2) comprise eight lighting groups (20), the first light sources (21) of said light groups being positioned along corresponding reference axes (R) that lie on a reference plane (53), substantially perpendicular to the optical axis (51) of said objective group, are radially oriented with respect to the optical axis (51) of said objective group and are equally spaced one another of an angle of 45°.

6. Apparatus, according to one or more of the previous claims, **characterised in that** said lighting means (2) comprise at least a second extended light source (22), positioned at the opening (52) of said objective group, in a space region external to said opening (52), said imaging means being configured to acquire third images (I₃) of the ocular fundus, illuminated by the light (L₂) emitted by said second light source, when said second light source is activated.

7. Apparatus, according to claim 6, **characterised in that** said second light source (22) is positioned at a relatively long distance from the optical axis (51) of said objective group, with respect to said first light sources (21).

8. Apparatus, according to one or more of the previous claims, **characterised in that** said lighting means (2) comprise at least a third extended light source (23), positioned internally to the optical field defined by the opening (52) of said objective group, said imaging means being configured to acquire fourth images (I₄) of the ocular fundus, illuminated by the light (L₃) emitted by said third light source, when said third light source is activated.

9. Apparatus, according to claim 8, **characterised in that** said third light source (23) is formed by projecting a light beam (L₃₁), emitted by a fourth light source (24), on a lens (320) of said objective group (32), said fourth light source being positioned at the opening (52) of said objective group, in a space region external to said opening (52).

10. Apparatus, according to claim 8, **characterised in that** said third light source (23) is formed by a light emitting device mounted on a supporting frame (321) that extends through the opening (52) of said objective group.

11. Apparatus, according to one of the claims from 6 to 7 and one of the claims from 8 to 10, **characterised in that** said control unit is configured to consecutively activate the first light sources (21) of at least a lighting group (20) and at least one of said second and third light sources (22, 23) according to at least a third activation time sequence, said imaging means being configured to acquire fifth images (I₅) of the ocular fundus, illuminated by the light (L₁, L₂, L₃) emitted by said first light sources (21) and by at least one of said first and second light sources (22, 23), activated according to said third activation time sequence, said control unit being configured to:
- receive said fifth images (I₅) from the imaging means (3);
- identify a front-edge of a crescent of light in said fifth images (I₅);
- calculate data related to the position and moving speed of said front-edge of said crescent of light in said fifth images (I₅);
- provide information (E) related to the type and extent of a sight defect on the base of the data calculated at the previous steps.

12. Apparatus, according to one or more of the previous claims, **characterised in that** said objective group (32) has a focal distance of 1 m.

## Patentansprüche

1. Vorrichtung (1) zum Erfassen von Augenschäden bei einem Patienten (100), aufweisend:
- eine Beleuchtungseinrichtung (2), die so konfiguriert ist, dass sie ein Licht (L₁, L₂, L₃) projiziert, um den Augenfundus zu beleuchten;
- eine Abbildungseinrichtung (3), die eine Erfassungseinrichtung (31) aufweist, die so konfiguriert ist, dass sie Licht, das von dem Augenfundus reflektiert wird, an einer Empfangsfläche (310) empfängt und so konfiguriert ist, dass sie Bilder (I₁, I₂, I₃, I₄, I₅) des Augenfundus erhält, wobei die Abbildungseinrichtung eine Objektivgruppe (32) aufweist, die eine optische Achse (51), die in Richtung der Augen des Patienten ausgerichtet ist, und eine Öffnung (52), die auf der optischen Achse zentriert ist, aufweist;
- eine Steuereinheit (4), die so konfiguriert ist, dass sie den Betrieb der Beleuchtungseinrichtung und der Abbildungseinrichtung steuert;
wobei die Beleuchtungseinrichtung (2) zumindest eine Beleuchtungsgruppe (20) aufweist, die eine Mehrzahl an ersten verlängerten Lichtquellen (21) aufweist, die an der Öffnung (52) der Objektivgruppe in einem Raumbereich, der sich außerhalb der Öffnung (52) befindet, entlang einer Referenzachse (R) in verschiedenen Entfernungen im Verhältnis zu der optischen Achse (51) der Objektivgruppe positioniert sind;
wobei die Steuereinheit (4) so konfiguriert ist, dass sie die ersten Lichtquellen gemäß zumindest einer ersten Aktivierungszeitsequenz nacheinander aktiviert, so dass der Augenfundus der Augen des Patienten gemäß einer vordefinierten Bewegungsrichtung so erleuchtet wird, als wäre er durch eine identische verlängerte Lichtquelle erleuchtet, die sich entlang der Referenzachse (R) bewegt;
wobei die Abbildungseinrichtung (3) so konfiguriert ist, dass sie erste Abbildungen (I₁) des Augenfundus erhält, der durch das Licht (L₁) erleuchtet wird, das durch jede der ersten Lichtquellen (21) emittiert wird, wenn die ersten Lichtquellen durch die Steuereinheit gemäß der ersten Aktivierungszeitsequenz aktiviert werden;
**dadurch gekennzeichnet, dass** die Steuereinheit (4) so konfiguriert ist, dass sie:
- die ersten Abbildungen (I₁) von der Abbildungseinrichtung (3) empfängt;
- eine Vorderkante einer Lichtsichel in den ersten Abbildungen (I₁) identifiziert;
- Daten berechnet, die sich auf die Position und Bewegungsgeschwindigkeit der Vorderkante der Lichtsichel in den ersten Abbildungen (I₁) beziehen;
- Informationen (E) zur Verfügung stellt, die sich auf den Typ und das Ausmaß einer Sichtbehinderung auf der Basis der in den vorhergehenden Schritten berechneten Daten beziehen.

2. Vorrichtung nach Anspruch 1, **dadurch gekennzeichnet, dass** die Beleuchtungsgruppe (20) zumindest drei erste Lichtquellen (21) aufweist, die entlang der Referenzachse (R) positioniert sind.

3. Vorrichtung nach einem oder mehreren der vorgenannten Ansprüche, **dadurch gekennzeichnet, dass** die ersten Lichtquellen (21) entlang der Referenzachse (R) positioniert sind, die auf einer Referenzebene (53) liegt, die im Wesentlichen rechtwinklig zu der optischen Achse (51) der Objektivgruppe ist und im Verhältnis zu der optischen Achse (51) der Objektivgruppe radial orientiert ist.

4. Vorrichtung nach einem oder mehreren der vorgenannten Ansprüche, **dadurch gekennzeichnet, dass** die Beleuchtungseinrichtung (2) eine Mehrzahl an Beleuchtungsgruppen (20) aufweist, die an unterschiedlichen Winkelpositionen im Verhältnis zu der optischen Achse (51) der Objektivgruppe positioniert sind, wobei die Steuereinheit so konfiguriert ist, dass sie die Beleuchtungsgruppen gemäß einer zumindest zweiten Aktivierungszeitsequenz nacheinander aktiviert, wobei die Abbildungseimichtung so konfiguriert ist, dass sie zweite Abbildungen (I₂) des Augenfundus erhält, der durch das Licht (L₁) beleuchtet wird, das durch jede der Beleuchtungsgruppen emittiert wird, wenn die Beleuchtungsgruppen gemäß der zweiten Aktivierungszeitsequenz aktiviert werden, wobei die Steuereinheit so konfiguriert ist, dass sie die zweiten Abbildungen (I₂) empfängt und verarbeitet und Informationen (E) zur Verfügung stellt, die sich auf das Vorhandensein von Augenschäden auf Basis von Daten, die durch die zweiten Abbildungen erhalten werden, beziehen.

5. Vorrichtung nach Anspruch 4, **dadurch gekennzeichnet, dass** die Beleuchtungseimichtung (2) acht Beleuchtungsgruppen (20) aufweist, wobei die ersten Lichtquellen (21) der Beleuchtungsgruppen entlang entsprechender Referenzachsen (R) positioniert sind, die auf einer Referenzebene (53) liegen und im Wesentlichen rechtwinklig zu der optischen Achse (51) der Objektivgruppe sind, im Verhältnis zu der optischen Achse (51) der Objektivgruppe radial ausgerichtet sind und zueinander in einem Winkel von 45° identisch beabstandet sind.

6. Vorrichtung nach einem oder mehreren der vorgenannten Ansprüche, **dadurch gekennzeichnet, dass** die Beleuchtungseinrichtung (2) zumindest eine zweite verlängerte Lichtquelle (22) aufweist, die an der Öffnung (52) der Objektivgruppe in einem Raumbereich außerhalb der Öffnung (52) positioniert ist, wobei die Abbildungseimichtung so konfiguriert ist, dass sie dritte Abbildungen (I₃) des Augenfundus erhält, der durch das Licht (L₂) beleuchtet wird, das durch die zweite Lichtquelle emittiert wird, wenn die zweite Lichtquelle aktiviert ist.

7. Vorrichtung nach Anspruch 6, **dadurch gekennzeichnet, dass** die zweite Lichtquelle (22) im Verhältnis zu den ersten Lichtquellen (21) in einer relativ großen Entfernung von der optischen Achse (51) der Objektivgruppe positioniert ist.

8. Vorrichtung nach einem oder mehreren der vorgenannten Ansprüche, **dadurch gekennzeichnet, dass** die Beleuchtungseinrichtung (2) zumindest eine dritte verlängerte Lichtquelle (23) aufweist, die im Inneren des durch die Öffnung (52) der Objektivgruppe definierten optischen Felds positioniert ist, wobei die Abbildungseimichtung so konfiguriert ist, dass sie vierte Abbildungen (I₄) des Augenfundus erhält, der durch das Licht (L₃) beleuchtet wird, das durch die dritte Lichtquelle emittiert wird, wenn die dritte Lichtquelle aktiviert ist.

9. Vorrichtung nach Anspruch 8, **dadurch gekennzeichnet, dass** die dritte Lichtquelle (23) durch Projizieren eines Lichtstrahls (L₃₁), der durch eine vierte Lichtquelle (24) emittiert wird, auf eine Linse (320) der Objektivgruppe (32) der vierten Lichtquelle, die an der Öffnung (52) der Objektivgruppe in einem Raumbereich, außerhalb der Öffnung (52) positioniert ist, gebildet wird.

10. Vorrichtung nach Anspruch 8, **dadurch gekennzeichnet, dass** die dritte Lichtquelle (23) durch eine Lichtemissionsvorrichtung gebildet wird, die auf einem Tragerahmen (321) montiert ist, der durch die Öffnung (52) der Objektivgruppe verläuft.

11. Vorrichtung nach einem der Ansprüche 6 bis 7 und einem der Ansprüche 8 bis 10, **dadurch gekennzeichnet, dass** die Steuereinheit so konfiguriert ist, dass sie die ersten Lichtquellen (21) von zumindest einer Beleuchtungsgruppe (20) und zumindest eine von den zweiten und dritten Lichtquellen (22, 23) gemäß einer zumindest dritten Aktivierungszeitsequenz nacheinander aktiviert, wobei die Beleuchtungseinrichtung so konfiguriert ist, dass sie fünfte Abbildungen (I₅) des Augenfundus erhält, der durch das Licht (L₁, L₂, L₃) beleuchtet wird, das von den ersten Lichtquellen (21) und zumindest einer von den ersten und zweiten Lichtquellen (22, 23) emittiert wird, die gemäß der dritten Aktivierungszeitsequenz aktiviert werden, wobei die Steuereinheit so konfiguriert ist, dass sie:
- die fünften Abbildungen (I₅) von der Abbildungseinrichtung (3) empfängt;
- eine Vorderkante einer Lichtsichel in den fünften Abbildungen (I₅) identifiziert;
- Daten berechnet, die sich auf die Position und Bewegungsgeschwindigkeit der Vorderkante der Lichtsichel in den fünften Abbildungen (I₅) beziehen;
- Informationen (E) zur Verfügung stellt, die sich auf den Typ und das Ausmaß einer Sichtbehinderung auf der Basis der in den vorhergehenden Schritten berechneten Daten beziehen.

12. Vorrichtung nach einem oder mehreren der vorgenannten Ansprüche, **dadurch gekennzeichnet, dass** die Objektivgruppe (32) eine Brennweite von 1m aufweist.

## Revendications

1. Appareil (1) pour détecter des défauts oculaires d'un patient (100) comprenant :
- des moyens d'éclairage (2) configurés pour projeter une lumière (L₁, L₂, L₃) pour éclairer le fond d'oeil ;
- des moyens de formation d'image (3) comprenant des moyens de détection (31) configurés pour recevoir la lumière réfléchie par le fond d'oeil, au niveau d'une surface de réception (310), et configurés pour acquérir des images (I₁, I₂, I₃, I₄, I₅) du fond d'oeil, lesdits moyens de formation d'image comprenant un groupe d'objectif (32) ayant un axe optique (51) dirigé vers les yeux du patient et une ouverture (52) centrée sur ledit axe optique ;
- une unité de commande (4) configurée pour commander le fonctionnement desdits moyens d'éclairage et desdits moyens de formation d'image ;
dans lequel lesdits moyens d'éclairage (2) comprennent au moins un groupe d'éclairage (20) comprenant une pluralité de premières sources de lumière étendues (21), positionnées au niveau de l'ouverture (52) dudit groupe d'objectif, dans une région spatiale à l'extérieur de ladite ouverture (52), le long d'un axe de référence (R), à différentes distances par rapport à l'axe optique (51) dudit groupe d'objectif ;
dans lequel ladite unité de commande (4) est configurée pour activer consécutivement lesdites premières sources de lumière conformément à au moins une première séquence d'instants d'activation de sorte que le fond d'oeil des yeux du patient soit éclairé comme s'il était éclairé par une même source de lumière étendue en mouvement le long de l'axe de référence (R), conformément à une direction de mouvement prédéfinie ;
dans lequel lesdits moyens de formation d'image (3) sont configurés pour acquérir des premières images (I₁) du fond d'oeil, éclairé par la lumière (L₁) émise par chacune desdites premières sources de lumière (21), lorsque lesdites premières sources de lumière sont activées par ladite unité de commande conformément à ladite première séquence d'instants d'activation ;
**caractérisé en ce que** ladite unité de commande (4) est configurée pour :
- recevoir lesdites premières images (I₁) des moyens de formation d'image (3) ;
- identifier un bord avant d'un croissant de lumière dans lesdites premières images (I₁) ;
- calculer des données relatives à la position et à la vitesse de déplacement dudit bord avant dudit croissant de lumière dans lesdites premières images (I₁) ;
- fournir des informations (E) relatives au type et à l'étendue d'un défaut de vue sur la base des données calculées aux étapes précédentes.

2. Appareil selon la revendication 1, **caractérisé en ce que** ledit groupe d'éclairage (20) comprend au moins trois premières sources de lumière (21), positionnées le long dudit axe de référence (R).

3. Appareil selon une ou plusieurs des revendications précédentes, **caractérisé en ce que** lesdites premières sources de lumière (21) sont positionnées le long dudit axe de référence (R), qui se trouve dans un plan de référence (53), sensiblement perpendiculaire à l'axe optique (51) dudit groupe d'objectif, et qui est orienté radialement par rapport à l'axe optique (51) dudit groupe d'objectif.

4. Appareil selon une ou plusieurs des revendications précédentes, **caractérisé en ce que** lesdits moyens d'éclairage (2) comprennent une pluralité de groupes d'éclairage (20), positionnés à différentes positions angulaires par rapport à l'axe optique (51) dudit groupe d'objectif, ladite unité de commande étant configurée pour activer consécutivement lesdits groupes d'éclairage conformément à au moins une deuxième séquence d'instants d'activation, lesdits moyens de formation d'image étant configurés pour acquérir des deuxièmes images (I₂) du fond d'oeil, éclairé par la lumière (L₁) émise par chacun desdits groupes d'éclairage, lorsque lesdits groupes d'éclairage sont activés conformément à ladite deuxième séquence d'instants d'activation, ladite unité de commande étant configurée pour recevoir et traiter lesdites deuxièmes images (I₂) et fournir des informations (E) relatives à la présence de défauts oculaires sur la base de données obtenues à partir desdites deuxièmes images.

5. Appareil selon la revendication 4, **caractérisé en ce que** lesdits moyens d'éclairage (2) comprennent huit groupes d'éclairage (20), les premières sources de lumière (21) desdits groupes d'éclairage étant positionnées le long d'axes de référence (R) correspondants qui se trouvent dans un plan de référence (53), sensiblement perpendiculaire à l'axe optique (51) dudit groupe d'objectif, qui sont orientés radialement par rapport à l'axe optique (51) dudit groupe d'objectif et qui sont régulièrement espacés les uns des autres d'un angle de 45°.

6. Appareil selon une ou plusieurs des revendications précédentes, **caractérisé en ce que** lesdits moyens d'éclairage (2) comprennent au moins une deuxième source de lumière étendue (22), positionnée au niveau de l'ouverture (52) dudit groupe d'objectif, dans une région spatiale à l'extérieur de ladite ouverture (52), lesdits moyens de formation d'image étant configurés pour acquérir des troisièmes images (I₃) du fond d'oeil, éclairé par la lumière (L₂) émise par ladite deuxième source de lumière, lorsque ladite deuxième source de lumière est activée.

7. Appareil selon la revendication 6, **caractérisé en ce que** ladite deuxième source de lumière (22) est positionnée à une distance relativement grande de l'axe optique (51) dudit groupe d'objectif, par rapport aux dites premières sources de lumière (21).

8. Appareil selon une ou plusieurs des revendications précédentes, **caractérisé en ce que** lesdits moyens d'éclairage (2) comprennent au moins une troisième source de lumière étendue (23), positionnée à l'intérieur du champ optique défini par l'ouverture (52) dudit groupe d'objectif, lesdits moyens de formation d'image étant configurés pour acquérir des quatrièmes images (I₄) du fond d'oeil, éclairé par la lumière (L₃) émise par ladite troisième source de lumière, lorsque ladite troisième source de lumière est activée.

9. Appareil selon la revendication 8, **caractérisé en ce que** ladite troisième source de lumière (23) est formée en projetant un faisceau de lumière (L₃₁), émis par une quatrième source de lumière (24), sur une lentille (320) dudit groupe d'objectif (32), ladite quatrième source de lumière étant positionnée au niveau de l'ouverture (52) dudit groupe d'objectif, dans une région spatiale à l'extérieur de ladite ouverture (52).

10. Appareil selon la revendication 8, **caractérisé en ce que** ladite troisième source de lumière (23) est formée par un dispositif d'émission de lumière monté sur un cadre de support (321) qui s'étend à travers l'ouverture (52) dudit groupe d'objectif.

11. Appareil selon l'une des revendications 6 et 7 et l'une des revendications 8 à 10, **caractérisé en ce que** ladite unité de commande est configurée pour activer consécutivement les premières sources de lumière (21) d'au moins un groupe d'éclairage (20) et au moins l'une desdites deuxièmes et troisièmes sources de lumière (22, 23) conformément à au moins une troisième séquence d'instants d'activation, lesdits moyens de formation d'image étant configurés pour acquérir des cinquièmes images (I₅) du fond d'oeil, éclairé par la lumière (L₁, L₂, L₃) émise par lesdites premières sources de lumière (21) et par au moins l'une desdites deuxièmes et troisièmes sources de lumière (22, 23), activées conformément à ladite troisième séquence d'instants d'activation, ladite unité de commande étant configurée pour :
- recevoir lesdites cinquièmes images (I₅) desdits moyens de formation d'image (3) ;
- identifier un bord avant d'un croissant de lumière dans lesdites cinquièmes images (I₅) ;
- calculer des données relatives à la position et à la vitesse de déplacement dudit bord avant dudit croissant de lumière dans lesdites cinquièmes images (I₅) ;
- fournir des informations (E) relatives au type et à l'étendue d'un défaut de vue sur la base des données calculées aux étapes précédentes.

12. Appareil selon une ou plusieurs des revendications précédentes, **caractérisé en ce que** ledit groupe d'objectif (32) a une distance focale de 1 m.
